# EUROPEAN PATENT APPLICATION

(11) **EP 1 911 490 A1**
(43) Date of publication of application: **16.04.2008**
(21) Application number: 06781204.0
(22) Date of filing: 18.07.2006
(51) Int. Cl.: A61N 1/36, A61N 1/04, A61N 1/05

(54) **SLEEP DISORDER TREATING DEVICE AND SLEEP DISORDER TREATING METHOD**

(30) Priority: 04.08.2005 JP 2005227125
(71) Applicant: Lintec Corporation, Tokyo 173-0001 (JP); Fes Co., Ltd., Aoba-ku Sendai-shi Miyagi 9800014 (JP)
(72) Inventor: YAMAGUCHI, Hiroshi c/o LINTEC CORPORATION, Tokyo 1730001 (JP); SASAKI, Hidemi c/o FES Co., Ltd., Sendai-shi, Miyagi; 9800014 (JP); SUZUKI, Kenji c/o FES Co., Ltd., Sendai-shi, Miyagi; 9800014 (JP); ENDO, Masakazu c/o FES Co., Ltd., Sendai-shi, Miyagi; 9800014 (JP); MORISHITA, Daisaburo c/o FES Co., Ltd., Sendai-shi, Miyagi; 9800014 (JP); HANDA, Yasunobu, Iwanuma-shi, Miyagi 9892433 (JP)
(74) Representative: Nicholls, Michael John
(86) International application number: PCT/JP2006/314191
(87) International publication number: WO 2007/015368

(57) **Abstract**

The sleep disorder therapeutic apparatus 1 comprises: four electrode pads 2 that can contact the body surface corresponding to the left and right second and third dorsal sacral foramina; a controller 3 that can supply pulse voltage to the electrode pads 2; and a harness 4 comprising a cover member 41 to which the four electrode pads 2 are attached, and a belt member 42 that can be wrapped around the abdomen such that the cover member 41 is tightly secured to the body surface.

## Description

### TECHNICAL FIELD

The present invention relates to a sleep disorder therapeutic apparatus and sleep disorder therapeutic method for treating sleep disorder by electrical stimulation.

### BACKGROUND ART

Generally, the number of nighttime arousal during sleep increases as one ages. Because sleep disorders caused by arousal during sleep prevent sound nighttime sleep, in some cases, daytime sleepiness becomes troublesome, there is a noticeable drop in activity leading to a drop in quality of life (QOL), and when severe, the symptoms progress to depression.

Nighttime sleep disorders in people living in nursing homes, elder care facilities, and the like are a serious problem, and the number of nighttime calls, that is, the number of arousal during sleep is an average of 5 to 6 times per person per night, further 10 times in the case of a serious person.

Meanwhile, the urinary disturbance therapeutic apparatus described in Japan Examined Patent Publication H3-26623 is well known, but neither the use of the related apparatus for sleep disorder therapy nor the use of electric stimulation for sleep disorder therapy has been known at all.

### DISCLOSURE OF THE INVENTION

With the foregoing in view, it is an object of the present invention to provide an apparatus and method effective for the treatment of sleep disorders.

To achieve the aforementioned object, first, the present invention provides a sleep disorder therapeutic apparatus that can impart electric stimulation to a site or nearby region of a nerve that controls the bladder (invention 1).

By using the sleep disorder therapeutic apparatus related to the aforementioned invention (invention 1), the urge to urinate that occurs frequently during the night can be suppressed, hence arousal during sleep can be prevented, and also disorder of initiating sleep can be improved. Specifically, according to the aforementioned invention (invention 1), it is possible to treat sleep disorders, and in particular, it is possible to effectively treat sleep disorders by arousal during sleep. Note that, "therapy" in the present invention means preventing or suppressing onset of sleep disorder while using the sleep disorder therapeutic apparatus related to the present invention, and does not mean preventing or suppressing onset of sleep disorder after discontinuing use of the sleep disorder therapeutic apparatus related to the present invention.

The sleep disorder therapeutic apparatus related to the aforementioned invention (invention 1) may comprise a pulse voltage generator that can generate pulse voltage, and at least one pair of electrodes that can apply pulse voltage from the voltage generator to a site or nearby region of the nerve that controls the bladder (invention 2).

In the aforementioned invention (invention 2), the electrodes may be electrode pads that contact the body surface corresponding to the dorsal sacral foramina (invention 3).

In the aforementioned invention (invention 3), preferably the sleep disorder therapeutic apparatus comprises a harness that can secure the electrode pads to the body, and the electrode pads are attached to the harness so as to contact the body surface corresponding to the left and right dorsal sacral foramina (invention 4). According to the related invention (invention 4), the electrode pads can be easily secured to the body.

In the aforementioned invention (invention 4), the electrode pads preferably are attached to the harness so as to contact the body surface corresponding to the left and right second and third dorsal sacral foramina (invention 5). According to the related invention (invention 5), it is possible to effectively conduct therapy because pulse voltage can be precisely applied to the nerve that controls the bladder.

The harness in the aforementioned inventions (inventions 4 and 5) preferably comprises a cover member that can cover the body surface from the lower back to the buttocks, and a belt member that can be wrapped around the body such that the cover member is tightly secured to the body surface and the electrode pads are attached to the cover member (invention 6). According to the related invention (invention 6), the electrode pads can be easily and reliably secured to the body.

In aforementioned invention (invention 6), preferably a positioning part to match the position on the body surface corresponding to the coccyx is provided on the cover member, and the electrode pads are attached to the cover member such that the electrode pads contact the body surface corresponding to the left and right dorsal sacral foramina by matching the position with the positioning part (invention 7). According to the related invention (invention 7), the electrode pads can be easily and precisely made to contact the body surface corresponding to the left and right dorsal sacral foramina.

In the aforementioned inventions (inventions 2 to 7), preferably the sleep disorder therapeutic apparatus comprises a control unit that can intermittently apply pulse voltage (invention 8). The related invention (invention 8) prevents the person being treated from becoming accustomed to the electric stimulation, and therapy can be conducted efficiently.

The aforementioned invention (invention 1) may comprise a stimulation element that is implanted in the body (invention 9). Moreover, in the aforementioned invention (invention 2), the electrodes may pierce into or be implanted in a site or nearby region of the nerve that controls the bladder (invention 10).

According to the aforementioned inventions (inventions 9 and 10), the therapeutic effect can be obtained at a lower voltage than with electrode pads because the stimulation elements or electrodes can be arranged in a region close to the nerve that controls the bladder.

Secondly, the present invention provides a sleep disorder therapeutic method wherein electric stimulation is applied to a site or nearby region of a nerve that controls the bladder (invention 11).

In the aforementioned invention (invention 11), preferably the aforementioned electric stimulation is applied to the dorsal sacral foramina or nearby (including the body surface corresponding to the dorsal sacral foramina) (invention 12).

In the aforementioned invention (invention 12), preferably the dorsal sacral foramina are the left and right second and third dorsal sacral foramina (invention 13).

In the aforementioned inventions (inventions 11 to 13), preferably the electric stimulation is electric stimulation based on pulse voltage (invention 14).

In the aforementioned invention (invention 14), preferably the pulse voltage is intermittently applied (invention 15).

In the aforementioned invention (invention 15), preferably the pulse voltage is applied for 3 to 10 seconds of application with an interval of 3 to 10 seconds of non-application (invention 16).

In the aforementioned inventions (inventions 14 to 16), preferably the pulse voltage is applied under the conditions of voltage 20 to 30V, current 10 to 40 mA, pulse width 100 to 400 µsec, and frequency 3 Hz to 10 kHz (invention 17).

In the aforementioned inventions (inventions 11 to 17), preferably the electric stimulation is conducted within 3 hours prior to going to bed, one time or more per day, 5 minutes or more each time, and in particular, 15 minutes or more (invention 18).

According to the present invention, it is possible to treat sleep disorders, and in particular, it is possible to effectively treat sleep disorders by arousal during sleep.

### BRIEF DESCRIPTION OF THE DRAWINGS

FIG. 1 is a perspective view seen from the front of a sleep disorder therapeutic apparatus related to a first embodiment of the present invention;
FIG. 2 is a back side view indicating the state of a sleep disorder therapeutic apparatus related to the same embodiment when attached;
FIG. 3 is a diagram indicating the state of a sleep disorder therapeutic apparatus related to a second embodiment of the present invention when in use; and
FIG. 4 is a diagram indicating the state of a sleep disorder therapeutic apparatus related to a third embodiment of the present invention when in use.

### BEST MODE FOR CARRYING OUT THE INVENTION

Embodiments of the present invention will be explained below.

### [First embodiment]

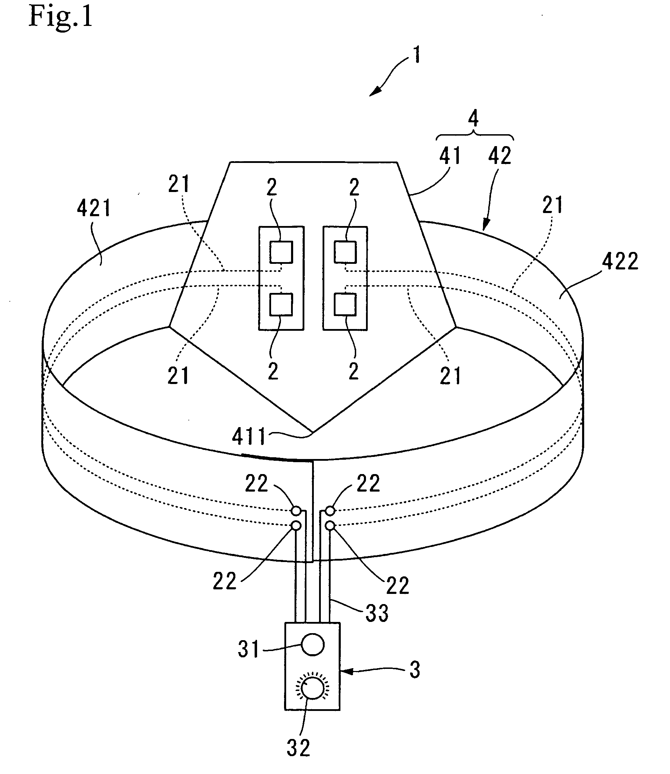

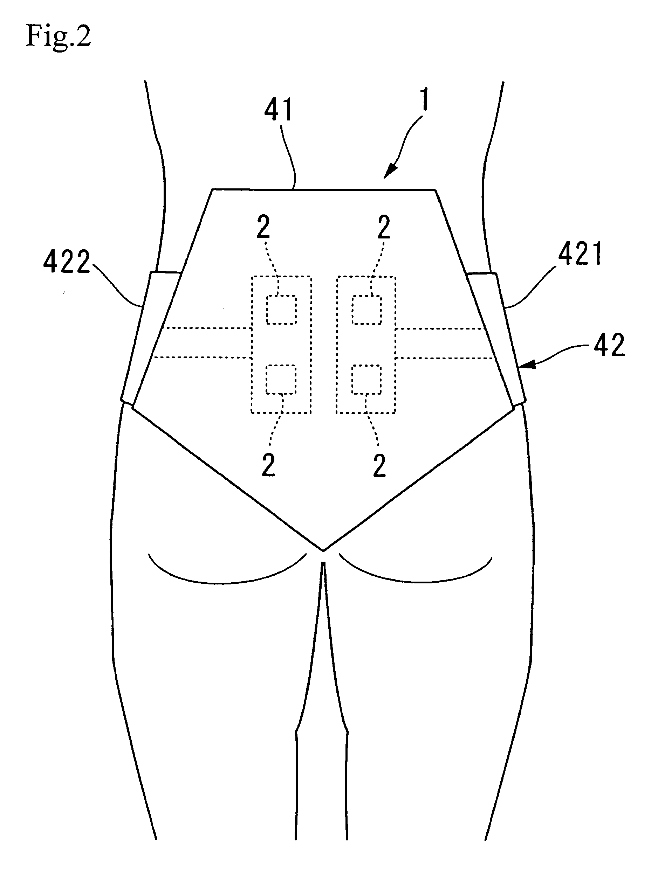

FIG. 1 is a perspective view seen from the front of a sleep disorder therapeutic apparatus related to a first embodiment of the present invention; and FIG. 2 is a back side view indicating the state of a sleep disorder therapeutic apparatus related to the same embodiment when attached.

As indicated in FIG. 1, a sleep disorder therapeutic apparatus 1 related to the present embodiment comprises four electrode pads 2, a controller 3 that can provide pulse voltage to the electrode pads 2, and a harness 4 that can secure the electrode pads 2 to a body.

The harness 4 comprises a cover member 41 that can cover a body surface from the lower back to the buttocks, and a belt member 42 that can be wrapped around the abdomen to tightly secure the cover member 41 to the body surface, and the four electrode pads 2 are attached to the cover member 41.

The material of the cover member 41 is not particularly limited as long as the electrode pads 2 can make precise contact with the body surface, and, for example, woven or non-woven cloth may be adopted.

In the present embodiment, the cover member 41 has an inverse pentagonal shape, and as indicated in FIG. 2, the each electrode pads 2 are arranged on the cover member 41 so that the four electrode pads 2 contact the body surface that corresponds the left and right second and third dorsal sacral foramina by matching the lowest vertex (lower edge part 411) of the cover member 41 to the coccyx.

The material of the electrode pads 2 is not particularly limited as long as pulse voltage can be precisely applied to the body, but, generally, a material that includes a water component and has low electrical resistance is preferable. Gel compositions of polyacrylic acid may be cited as preferable related materials. In particular, a substance comprising partially neutralized polyacrylic acid (main component) 4.50 to 5.70 mass%, polyacrylic acid (main component) 0.00 to 4.00 mass%, concentrated glycerin (moisturizer) 34.88 to 55.22 mass%, dihydroxyaluminum aminoacetate (cross-linking agent) 0.13 to 0.18 mass%, methylparaben (preservative) 0.13 to 0.18 mass%, and purified water (water component) 40.00 to 55.00 mass% may be cited as a preferable gel composition of polyacrylic acid.

Belt member 42 comprises a right belt member 421 and a left belt member 422, which are respectively attached to the right and left of cover member 41. The right belt member 421 and the left belt member 422 can be mutually fastened using a surface fastener such as magic tape (registered trademark).

Lead lines 21 of the two electrode pads 2 for the right dorsal sacral foramina are provided to follow along the right belt member 421, and to reach to connectors 22 provided on the front side of the right belt member 421; and lead lines 21 of the two electrode pads 2 for the left dorsal sacral foramina are provided to follow along the left belt member 422, and to reach to the connectors 22 provided on the front side of the left belt member 422.

Meanwhile, the controller 3 is provided with a pulse voltage generator (not indicated in the figure) that can generate pulse voltage, a voltage volume 32 that can manually adjust the level of the pulse voltage applied, a power source to drive the pulse voltage generator (not indicated in the figure), a switch 31 that can turn the power source ON/OFF, and a control unit that automatically controls the power source to turn ON/OFF at a specified interval (not indicated in the figure).

Four lead lines 33 that are connected to the pulse voltage generator proceed from the controller 3, and can connect to the respective connectors 22 of the belt member 42. By connecting these lead lines 33 to the connectors 22, the pulse voltage generator and the electrode pads 2 are electrically connected.

The pulse voltage generator may comprise a conventional well-known circuit, and is not particularly limited (for example, refer to the stimulation apparatus in Japan Examined Patent Publication H3-26623). The pulse width of the pulse voltage generated is 10 to 500 µsec, preferably 100 to 400 µsec, and more preferably 200 to 300 µsec. The frequency of the pulse voltage generated is 1 Hz to 100 kHz, preferably 3 Hz to 10 kHz, and more preferably 3 Hz to 50 Hz.

Preferably, the pulse voltage can be adjusted in the range of at least 22 to 30 V by the voltage volume 32. The preferable range of adjustment is 0 to 120 V, and in particular, 0 to 80 V. At that time, the current is preferably 10 to 50 mA, and in particular, about 10 to 40 mA.

Preferably, the control unit automatically conducts control such that the power source is repeatedly turned ON/OFF with the power source ON for 3 to 10 seconds and the power source OFF for 3 to 10 seconds. By intermittently applying the pulse voltage to the person receiving therapy, that person can thereby be prevented from becoming accustomed to the electric stimulation, and the therapy can be conducted efficiently. Note that, the control unit can be comprised by a conventional well-known circuit, and is not particularly limited.

The controller 3 may have a timer function that turns the power source from OFF to ON after a specified time has elapsed. In this case, one or more specified time may be selected from, for example, 5 minutes, 10 minutes, 15 minutes, 20 minutes or 30 minutes.

To use the sleep disorder therapeutic apparatus 1 described above, first, the cover member 41 is positioned on the lower back and buttocks by aligning the lower edge part 411 of the cover member 41 on the coccyx, then the right belt member 421 and the left belt member 422 are wrapped around the abdomen and are fasted with a surface fastener. By attaching the harness 4 in this way, the four electrode pads 2 are reliably secured to the body surface corresponding to the left and right second and third dorsal sacral foramina.

Next, the switch 31 of the controller 3 is turned ON, the voltage volume 32 is adjusted, and the pulse voltage is increased to a level that is not painful. After left in that state for about 5 to 20 minutes, the switch 31 of the controller 3 is turned OFF. By using the related sleep disorder therapeutic apparatus 1 once per day or more, preferably within 3 hours prior to going to bed, arousal during sleep can be notably reduced, and depending on the case, disorder of initiating sleep can be improved. In this way, it is possible to treat sleep disorders with the sleep disorder therapeutic apparatus 1, and in particular, sleep disorders by arousal during sleep can be effectively treated.

Although the mechanism is not completely understood, applying pulse voltage to the vicinity of the second and third dorsal sacral foramina (site of the nerve that controls the bladder) stimulates the nerve that controls the bladder and has some kind of further effect on the sympathetic and parasympathetic nervous systems that thereby appears to prevent arousal during sleep by suppressing the urge to urinate that frequently occurs during the night, and to improve disorder of initiating sleep. Note that, because the amount of urine in the bladder of the person receiving treatment prior to going to bed does not change whether or not treatment is conducted, it does not mean that arousal during sleep is prevented by augmenting urination prior to going to bed.

### [Second embodiment]

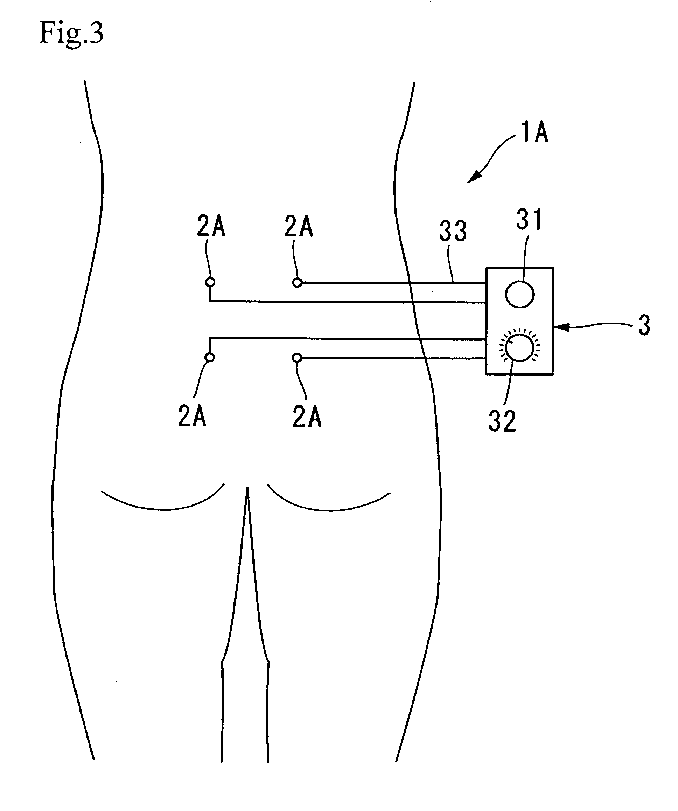

FIG. 3 is a diagram indicating the state of a sleep disorder therapeutic apparatus related to a second embodiment of the present invention when in use.

As indicated in FIG. 3, a sleep disorder therapeutic apparatus 1A related to the present embodiment is comprised of four electrodes 2A, and a controller 3 that can supply pulse voltage to the electrodes 2A.

The electrodes 2A of the present embodiment are implanted or inserted near a site of the nerve that controls the bladder, preferably, in the left and right second and third dorsal sacral foramina, and, for example, may be selected from needle-shaped objects, stranded wire-shaped objects, and the like.

The rear terminals of electrodes 2A, or lead lines connected to electrodes 2A, exit the body, and lead lines 33 of the controller 3 connect thereto. The same unit as that in the first embodiment may be used as the controller 3, but therapy can be effectively conducted at a lower voltage because the electrode pads 2A can be arranged at a site nearer to the nerve that controls the bladder than is possible with the electrode pads 2.

Specifically, the voltage volume 32 preferably can adjust the pulse voltage at least in the range of 5 to 15 V. The preferable range of adjustment is 0 to 20 V.

### [Third embodiment]

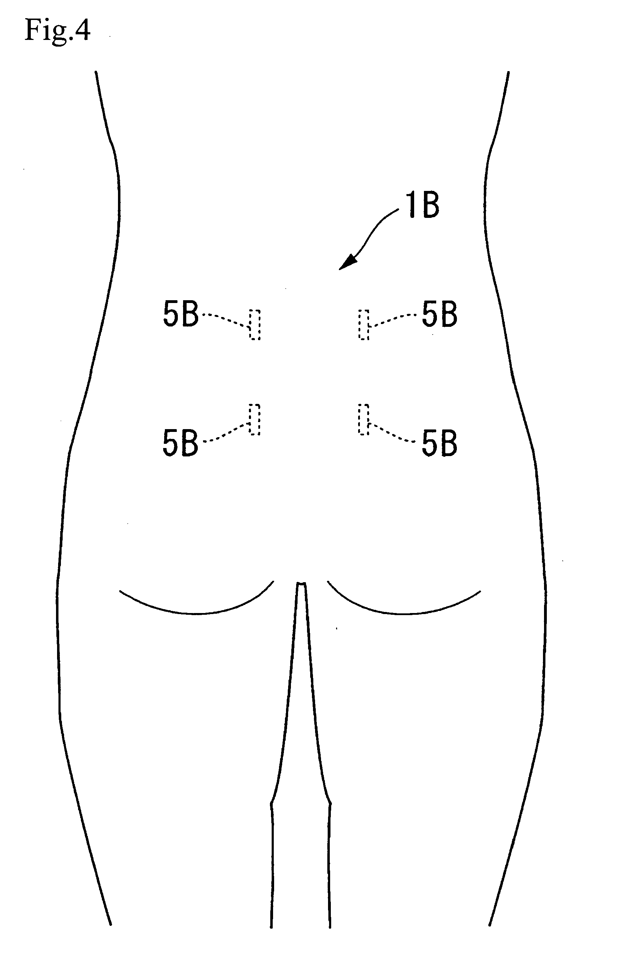

FIG. 4 is a diagram indicating the state of a sleep disorder therapeutic apparatus related to a third embodiment of the present invention when in use.

As indicated in FIG. 4, the sleep disorder therapeutic apparatus 1B related to the present embodiment comprises four stimulation elements 5B implanted near a site of the nerve that controls the bladder, preferably, at the left and right second and third dorsal sacral foramina.

The stimulation elements 5B may be a type that comprises electrodes and a receiver function, and is controlled by an external transmitter, a type that produces electricity based on an externally imparted magnetic field, or a type that comprises an independent power source itself. For any of the types, conventional well-known apparatuses can be utilized.

In the present embodiment as well, therapy can be effectively conducted at a lower voltage because the electric stimulation can be imparted at a site nearer to the nerve that controls the bladder than is possible with the electrode pads 2 in the first embodiment.

The embodiments explained above have been described in order to make the present invention easy to understand, and were not described in order to limit the present invention. Consequently, it is intended that the essential elements disclosed in the previously described embodiments include all design modifications and equivalent devices belonging to the technical range of the present invention.

For example, instead of four, two or six or more of the electrode pads 2, electrodes 2A or stimulation elements 5B may be comprised respectively.

### [Therapeutic method]

The sleep disorder therapeutic method of the present invention applies electric stimulation of pulse voltage and the like to a site or nearby region of the nerve that controls the bladder, preferably the dorsal sacral foramina or nearby (including the body surface corresponding to the dorsal sacral foramina), and more particularly, to the left and right second and third dorsal sacral foramina or nearby (including the body surface corresponding to the left and right second and third dorsal sacral foramina).

The aforementioned sleep disorder therapeutic method can be preferably conducted using the sleep disorder therapeutic apparatus related to the previously described embodiments, but it is not always necessary to use these apparatuses.

Pulse voltage is preferably used as the electric stimulation. The pulse voltage is preferably applied intermittently, and in particular, is intermittently applied using 3 to 10 seconds of application with an interval of 3 to 10 seconds of non-application.

Moreover, the pulse voltage is preferably applied under the following conditions:
Voltage: 20 to 30 V
Current: 10 to 40 mA
Pulse width: 100 to 400 µsec
Frequency:3 Hz to 10 kHz

The application of the electric stimulation (pulse voltage) is preferably conducted within 3 hours of going to bed, once or more per day for 5 minutes or more each time, in particular, 15 minutes or more.

According to the above sleep disorder apparatus, arousal during sleep can be notably reduced, and depending on the case, disorder of initiating sleep can be improved.

### EXAMPLES

### [Example 1]

The sleep disorder therapeutic apparatus indicated in FIG. 1 was fabricated using a low frequency therapeutic device "Nodoka" manufactured by Lintec. Eight persons were targeted who were admitted to an elder nursing care facility, free of bladder and urinary tract infections and the like, and who had sleep disorders caused by arousal during sleep because of the frequent onset of nighttime urinary urge while having a normal nighttime urinary volume. Treatment by the aforementioned sleep disorder therapeutic apparatus was conducted over 8 weeks for 15 minutes once per day prior to going to bed.

The conditions for applying the pulse voltage were as follows:
Pulse width: 200 µsec
Frequency:30 Hz
Voltage: Maximum value between 0 and 80 V at which the person receiving treatment does not feel pain
Current: 40 mA
Intermission timing: ON 5 sec/OFF 5 sec

Indicated in Table 1 are the results of counting the number of nighttime arousal during sleep and the number of nighttime calls prior to treatment by the aforementioned therapy, on the first day of treatment, after 4 weeks, and after 8 weeks from the start of the treatment, for each person receiving treatment.

**[Table 1]**

| | Person receiving treatment | A | B | C | D | E | F | G | H | Average |
|---|---|---|---|---|---|---|---|---|---|---|
| Number of nighttime arousal during sleep | Prior to treatment | 8 | 9 | 6 | 7 | 6 | 8 | 9 | 6 | 7.4 |
| | First day of treatment | 2 | 2 | 1 | 2 | 2 | 2 | 1 | 2 | 1.8 |
| | After 4 weeks | 2 | 2 | 1 | 2 | 1 | 2 | 1 | 2 | 1.6 |
| | After 8 weeks | 1 | 2 | 1 | 1 | 1 | 1 | 1 | 2 | 1.3 |
| | Prior to treatment | 8 | 10 | 5 | 7 | 8 | 8 | 9 | 7 | 7.8 |
| Number of nighttime calls | First day of treatment | 2 | 2 | 1 | 2 | 2 | 2 | 1 | 2 | 1.7 |
| | After 4 weeks | 2 | 2 | 0 | 2 | 1 | 2 | 1 | 2 | 1.3 |
| | After 8 weeks | 1 | 2 | 0 | 0 | 0 | 1 | 1 | 1 | 0.7 |

As is clear from Table 1, with the aforementioned therapy, nighttime arousal during sleep was notably reduced, and the number of nighttime calls decreased. Concretely, all the persons receiving treatment had reduced daytime activity caused by accumulated lack of sleep, because of awakening nearly every other hour through the night with the urge to urinate, and a severe case complained of symptoms of depression. However, the therapeutic effects were seen from the first night the aforementioned therapy was conducted, and the number of arousal during sleep fell to 1 to 2 times, and the sleep disorder was notably improved. Moreover, there was also a person who would calm down, have a drop in heart rate and become sleepy about 5 minutes after beginning stimulation by pulse voltage.

### [Example 2]

The same therapy as in the example 1 was conducted on 40 persons in an elder care home. The number of nighttime arousal during sleep and the number of nighttime calls were counted for each person receiving treatment prior to treatment, on the first day of treatment, after 1 week, after 2 weeks, after 4 weeks, and after 8 weeks from the start of the treatment, and the average numbers of the 40 people were calculated. Moreover, based on subjective symptoms, the degree of sleep disorder for each of the persons receiving treatment was assessed into the 3 stages of: mild (when mild daytime sleepiness occurred the day after because of nighttime arousal during sleep), moderate (when sleeping occurred the day after because of nighttime arousal during sleep), and severe (when the motivation to be active diminishes so that there was substantially no desire to move the day after because of nighttime arousal during sleep). The results are indicated in Table 2.

**[Table 2]**

| | Average number of nighttime arousal during sleep (times/person) | Average number of nighttime calls (times/person) | Mild sleep disorder (Number of cases) | Moderate sleep disorder (Number of cases) | Severe sleep disorder (Number of cases) |
|---|---|---|---|---|---|
| Prior to treatment | 8 | 6 | 2 | 25 | 13 |
| First day of treatment | 2 | 2 | 10 | 22 | 8 |
| After 1 week | 2 | 2 | 29 | 11 | 0 |
| After 2 weeks | 2 | 2 | 33 | 7 | 0 |
| After 4 weeks | 2 | 1 | 37 | 3 | 0 |
| After 8 weeks | 1 | 1 | 38 | 2 | 0 |

As is clear from Table 2, with the aforementioned therapy, nighttime arousal during sleep was notably reduced, and the number of nighttime calls decreased. Moreover, the sleep disorder became mild in 38 of the 40 persons receiving treatment and notable improvement of the sleep disorder was observed.

### INDUSTRIAL APPLICABILITY

The present invention is extremely useful for the treatment of sleep disorders, especially sleep disorders caused by nighttime arousal during sleep.

## Claims

1. A sleep disorder therapeutic apparatus that can impart electric stimulation to a site or nearby region of a nerve that controls the bladder.

2. The sleep disorder therapeutic apparatus according to claim 1 comprising:
a pulse voltage generator that can generate pulse voltage, and
at least one pair of electrodes that can apply pulse voltage from said voltage generator to a site or nearby region of the nerve that controls the bladder.

3. The sleep disorder therapeutic apparatus according to claim 2 wherein said electrodes are electrode pads that contact the body surface corresponding to the dorsal sacral foramina.

4. The sleep disorder therapeutic apparatus according to claim 3, further comprising a harness that can secure said electrode pads to the body, wherein said electrode pads are attached to said harness so as to contact the body surface corresponding to the left and right dorsal sacral foramina.

5. The sleep disorder therapeutic apparatus according to claim 4, wherein said electrode pads are attached to said harness so as to contact the body surface corresponding to the left and right second and third dorsal sacral foramina.

6. The sleep disorder therapeutic apparatus according to claim 4 or 5, wherein
the harness comprises a cover member that can cover the body surface from the lower back to the buttocks, and a belt member that can be wrapped around the body such that the cover member is tightly secured to the body surface, and
said electrode pads are attached to said cover member.

7. The sleep disorder therapeutic apparatus according to claim 6, wherein
a positioning part to match the position on the body surface corresponding to the coccyx is provided on said cover member, and
said electrode pads are attached to said cover member such that said electrode pads contact the body surface corresponding to the left and right dorsal sacral foramina by matching the position with said positioning part.

8. The sleep disorder therapeutic apparatus according to any of claims 2 to 7, further comprising a control unit that can intermittently apply pulse voltage.

9. The sleep disorder therapeutic apparatus according to claim 1, further comprising a stimulation element that is implanted in the body.

10. The sleep disorder therapeutic apparatus according to claim 2, wherein said electrode pierces into or is implanted in a site or nearby region of the nerve that controls the bladder.

11. A sleep disorder therapeutic method wherein electric stimulation is applied to a site or nearby region of a nerve that controls the bladder.

12. The sleep disorder therapeutic method according to claim 11, wherein said electric stimulation is applied to the dorsal sacral foramina or nearby.

13. The sleep disorder therapeutic method according to claim 12, wherein said dorsal sacral foramina are the left and right second and third dorsal sacral foramina.

14. The sleep disorder therapeutic method according to any of claims 11 to 13, wherein said electric stimulation is electric stimulation based on pulse voltage.

15. The sleep disorder therapeutic method according to claim 14, wherein said pulse voltage is intermittently applied.

16. The sleep disorder therapeutic method according to claim 15, wherein said pulse voltage is applied for 3 to 10 seconds of application with an interval of 3 to 10 seconds of non-application.

17. The sleep disorder therapeutic method according to any of claims 14 to 16, wherein said pulse voltage is applied under the conditions of voltage 20 to 30V, current 10 to 40 mA, pulse width 100 to 400 µsec, and frequency 3 Hz to 10 kHz.

18. The sleep disorder therapeutic method according to any of claims 11 to 17, wherein electric stimulation is conducted within 3 hours prior to going to bed, one time or more per day, and 5 minutes or more each time.
